# EUROPEAN PATENT APPLICATION

(11) **EP 3 607 963 A1**
(43) Date of publication of application: **12.02.2020**
(21) Application number: 18781748.1
(22) Date of filing: 06.04.2018
(51) Int. Cl.: A61K 38/16, A61P 1/00, A61P 1/16, A61P 1/18, A61P 9/00, A61P 11/00, A61P 13/12, A61P 15/00, A61P 17/00, A61P 17/02, A61P 19/00, A61P 19/02, A61P 19/04, A61P 21/00, A61P 27/02

(54) **THERAPEUTIC MEDICINE FOR FIBROUS DISEASE**

(30) Priority: 07.04.2017 JP 2017076600
(71) Applicant: Stemrim Inc., Ibaraki-shi, Osaka 567-0085 (JP); Osaka University, Suita-shi, Osaka 565-0871 (JP)
(72) Inventor: TAMAI, Katsuto, Suita-shi Osaka 565-0871 (JP); AOTO, Takahiro, 7-chome, Ibaraki-shi, Osaka 5670085 (JP); YAMAZAKI, Sho, Ibaraki-shi Osaka 567-0085 (JP); YAMAZAKI, Takehiko, Ibaraki-shi Osaka 567-0085 (JP)
(74) Representative: Zacco GmbH
(86) International application number: PCT/JP2018/014662
(87) International publication number: WO 2018/186480

(57) **Abstract**

The present inventors discovered that an HMGB1 fragment peptide having a particular amino acid sequence exhibits effects of inhibiting finger adhesion and digestive tract scarring and prolonging survival in the dystrophic epidermolysis bullosa model mice. Also in the skin ulcer model, administration of the specific HMGB1 fragment peptide was found to inhibit the fibrosing of skin during the healing process of the ulcer. Based on these findings, the present application provides pharmaceutical compositions for the treatment of fibrotic diseases, which comprise the specific HMGB1 fragment peptide.

## Description

### Technical Field

The present application relates to pharmaceutical compositions for treating fibrotic diseases, which comprise a fragment peptide of the HMGB1 (High Mobility Group Box 1) protein.

### Background Art

Fibrotic diseases represented by fibrosis of various organs and tissues are diseases in which excessive production and deposition of extracellular matrix proteins occur in various internal organs, organs, and tissues, such as the lung and liver, resulting in tissue hardening and dysfunction. Progression of tissue fibrosing in fibrotic diseases is often irreversible and currently there is no effective therapy, despite that the fibrotic disease is a serious disease that may lead to death when it progresses like in the case of pulmonary fibrosis, liver cirrhosis, or such.

Epidermolysis bullosa is an inheritable and intractable blistering skin disease in which the adhesion function between the epidermis and dermis is disrupted by genetic abnormality of adhesion structure regulatory proteins in the skin basement membrane region, and the epidermis is detached at the basement membrane level by slight external force in daily life to form blisters and ulcers like whole-body burn. For example, in dystrophic epidermolysis bullosa, loss or mutation of the Type VII Collagen α1 (COL7A1) gene disrupts the adhesion function between the basement membrane and dermis, causing repeated epidermolysis, blister formation, and healing, which results in increased fibrosing in the dermis and scar formation. However, a safe and reliable methodology to normalize genetic abnormalities is not established at the present, and there is no definitive therapy for hereditary diseases including epidermolysis bullosa.

### Citation List

### [Patent Document]

Patent Document 1: WO2012/147470
Patent Document 2: WO2014/065347

### [Non-Patent Document]

Non-Patent Document 1: Fritsch, et al., J Clin Invest. 2008 May; 118(5):1669-79

### Summary of the Invention

### [Problems to be Solved by the Invention]

An objective of the present application is to provide new pharmaceuticals effective for the treatment of fibrotic diseases including fibrosis of various organs.

### [Means for Solving the Problems]

As a result of searching for substances effective for the treatment of dystrophic epidermolysis bullosa which secondarily causes fibrosing of tissue, the present inventors found that an HMGB1 fragment peptide having a particular amino acid sequence exhibits an effect of inhibiting finger adhesion and scarring of the digestive tract, and an effect of prolonging the survival time in dystrophic epidermolysis bullosa model mice. The present inventors further found that administration of the specific HMGB1 fragment peptide inhibits fibrosing of skin during the healing process of ulcers in the skin ulcer model, which is not a genetic disorder such as epidermolysis bullosa, in which physical skin defects were created in mice that do not have genetic abnormalities. Based on these findings, the present application provides pharmaceutical compositions comprising the specific HMGB1 fragment peptide for treatment of fibrotic diseases.

That is, the present application provides the following:
[1] A pharmaceutical composition for treating a fibrotic disease, which comprises the substance described in any of (a) to (c) below (herein below referred to as substance A):
   (a) an HMGB1 fragment peptide comprising the amino acid sequence described in SEQ ID NO: 1;
   (b) a peptide comprising an amino acid sequence in which one or more amino acids have been substituted, deleted, inserted, or added in the amino acid sequence described in SEQ ID NO:1; and
   (c) a peptide comprising an amino acid sequence having about 80 % or more sequence identity with the amino acid sequence described in SEQ ID NO: 1.
[2] The pharmaceutical composition of [1], wherein the fibrotic disease is selected from the group consisting of systemic scleroderma, localized scleroderma, keloids, hypertrophic scars, scars after skin wounding or skin ulceration, skin fibrosis, myocardial fibrosis, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints.
[3] A pharmaceutical composition for inhibiting fibrosing of a tissue, which comprises substance A.
[4] An agent for inhibiting fibrosing of a tissue, which comprises substance A.
   [A1] A method of treating a fibrotic disease, which comprises administering an effective amount of substance A to a subject.
   [A2] The method of [A1], wherein the fibrotic disease is selected from the group consisting of systemic scleroderma, localized scleroderma, keloids, hypertrophic scars, scars after skin wounding or skin ulceration, skin fibrosis, myocardial fibrosis, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints.
   [A3] A method of inhibiting fibrosing of a tissue, which comprises administering an effective amount of substance A to a subject.
   [B1] Substance A for use in the treatment of a fibrotic disease.
   [B2] Substance A of [B1], wherein the fibrotic disease is selected from the group consisting of systemic scleroderma, localized scleroderma, keloids, hypertrophic scars, scars after skin wounding or skin ulceration, skin fibrosis, myocardial fibrosis, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints.
   [B3] Substance A for use in inhibiting fibrosing of a tissue.
   [C1] Use of substance A in the manufacture of a medicament for the treatment of a fibrotic disease.
   [C2] The use of [C1], wherein the fibrotic disease is selected from the group consisting of systemic scleroderma, localized scleroderma, keloids, hypertrophic scars, scars after skin wounding or skin ulceration, skin fibrosis, myocardial fibrosis, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints.
   [C3] Use of substance A in the manufacture of a medicament for inhibiting fibrosing of a tissue.
   [C4] Use of substance A in the manufacture of an agent for inhibiting fibrosing of a tissue.

### Brief Description of the Drawings

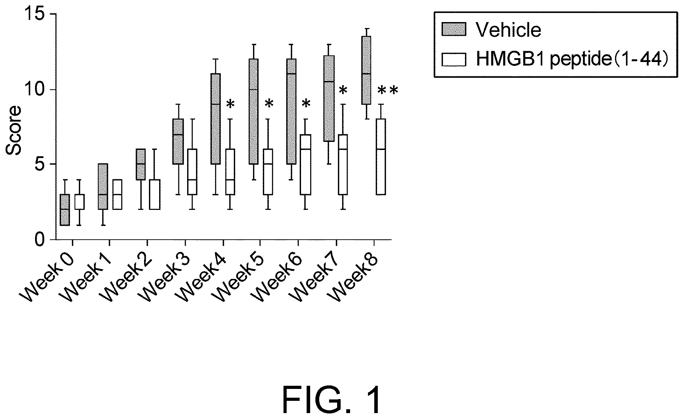

FIG. 1 is a graph showing adhesion scores of the HMGB1 peptide (1-44)-treated group and the vehicle group. The median, first quartile, third quartile, and data range are shown for each group (n = 5-7). * p<0.05 and ** p<0.01 indicate significant difference from the vehicle group (Mann-Whitney's U test).

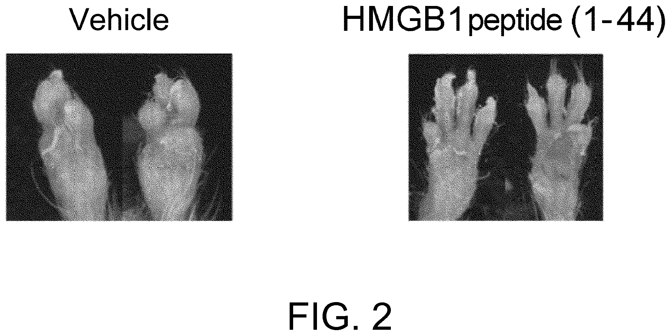

FIG. 2 is a photograph showing a representative example of the right and left forelimbs of mice six weeks after the start of administration.

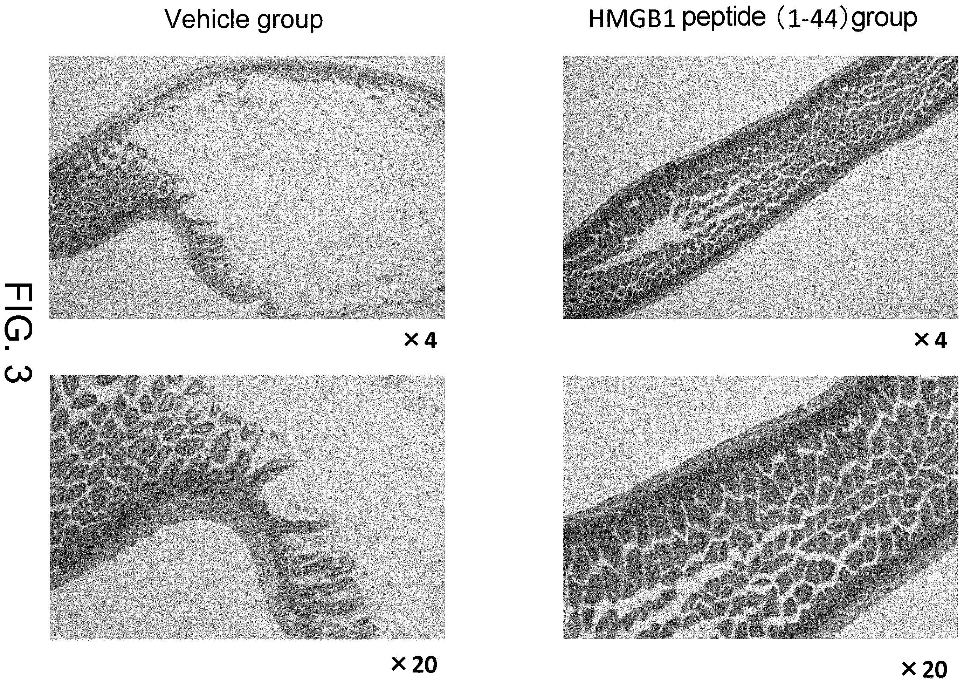

FIG. 3 is an image showing a representative example of the results of hematoxylin-eosin (HE) staining of the small intestines of mice ten weeks after the start of administration.

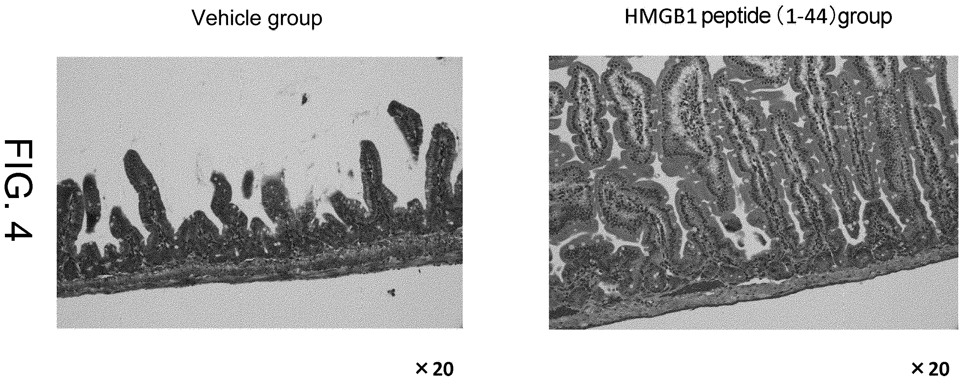

FIG. 4 is an image showing a representative example of the results of Masson's-Trichrome (MT) staining of the small intestines of mice eleven weeks after the start of administration. In the vehicle group, the area surrounding crypt cells are strongly stained with blue, and deposition of collagen fibers is confirmed. On the other hand, in the HMGB1 peptide (1-44)-treated group, blue-stained area is almost not seen.

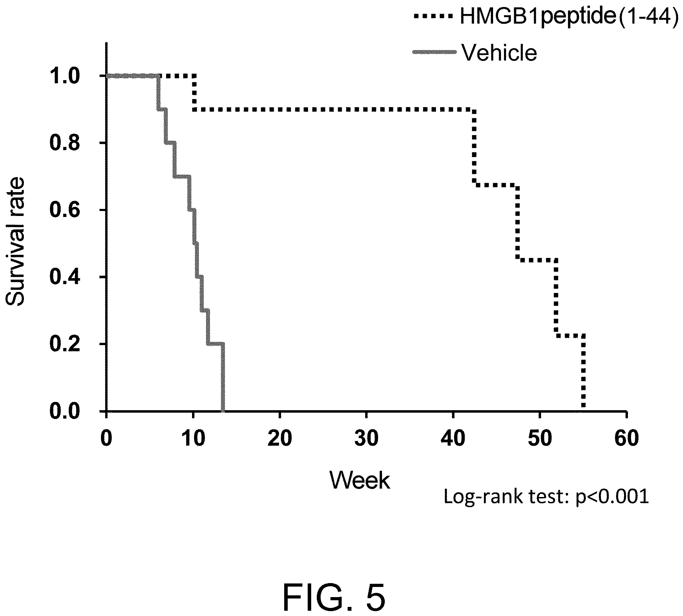

FIG. 5 is a graph showing the survival rate of the epidermolysis bullosa model mice (Kaplan-Meier curves). The vertical axis indicates survival rate (1.0 = 100%) and the horizontal axis indicates time (weeks) (Week 0 at the start of administration of the test substance). In the Log-rank test, there were significant differences between the groups (p<0.001).

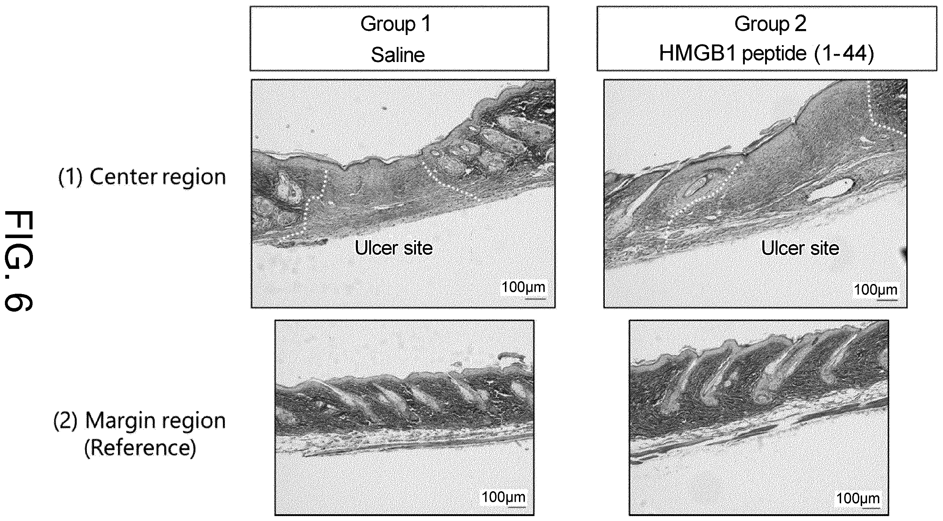

FIG. 6 is an image showing a representative example of Masson's-Trichrome staining of mouse skin 28 days after creation of skin ulcer. The blue-stained regions are collagen fibers, and the red-stained regions are mainly hematological cells, epithelial tissues, subcutaneous fascia, and such. The ulcer site (regenerated and healed) is the region within the dotted lines. Center region = ulcer region in the section. Margin region = non-ulcer site at the ulcer margin (used as reference for quantitative analysis).

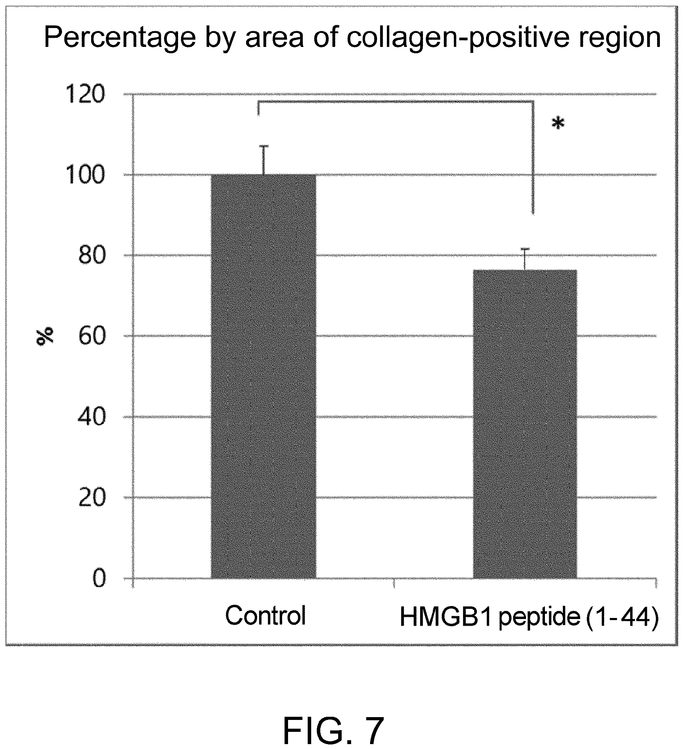

FIG. 7 is a graph showing the result of analyzing the percentage by area of collagen-positive region in the mouse skin 28 days after creation of skin ulcer. Data are presented as mean ± standard deviation (N=9) (*p<0.05, Mann Whitney's test).

### Mode for Carrying Out the Invention

The present application provides pharmaceutical compositions for the treatment of fibrotic diseases, which comprise an HMGB1 fragment peptide comprising the amino acid sequence described in SEQ ID NO: 1.

In the present application, "fibrotic disease" refers to a disease in which overproduction or deposition of extracellular matrix proteins occurs in an internal organ, organ, tissue, or the like, resulting in tissue hardening or dysfunction. Extracellular matrix proteins associated with fibrogenesis include collagen (e.g., Type I, III, IV, V, and VI collagens). The "fibrotic disease" in the present application includes, but is not limited to, systemic scleroderma, localized scleroderma, keloids, myocardial fibrosis, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis (e.g., cystic fibrosis of the pancreas), digestive tract scars, adipose tissue scars, scars in dystrophic epidermolysis bullosa, and postoperative scars of the skin, digestive tract, peritoneum, muscles, tendons, or joints. The "fibrotic disease" in the present application includes idiopathic and secondary diseases.

Pulmonary fibrosis is a disease in which collagen fibers deposit mainly in the interstitium of the lung, and the whole lung hardens, resulting in failure in the gas exchange function. Pulmonary fibrosis includes idiopathic pulmonary fibrosis and secondary pulmonary fibrosis.

Renal fibrosis is a disease in which collagen fibers deposit mainly in the interstitium of the kidneys, resulting in failure in the renal function.

Liver fibrosis is a disease in which collagen fibers deposit mainly in the liver, and the liver hardens, resulting in failure in the liver function. Liver cirrhosis is a condition in which collagen deposition and hardening of the liver further progress from liver fibrosis, and destruction of the hepatic lobule structure and formation of regenerative nodules are observed.

In the present application, "fibrogenesis/fibrosing" refers to the deposition of extracellular matrix proteins (primarily collagen) in tissues of the body. In the present application, "fibrosis" refers to a condition in which fibrosing of the body tissue has reached a level that results in hardening or dysfunction of the tissue. In the present application, "scarring" (also referred to as scar formation) is replacement of the damaged site in a body tissue by an extracellular matrix (primarily collagen fibers). In the present application, "scar" refers to a condition in which the damaged site in a body tissue is replaced by an extracellular matrix (primarily collagen fibers), or alternatively to a site in the damaged tissue that is replaced by an extracellular matrix (mainly collagen fibers).

In the present application, the term "pharmaceutical composition" is used interchangeably with "pharmaceutical", "drug", and "pharmacological composition".

In one embodiment, the fibrotic disease that can be treated using the pharmaceutical composition of the present application is:
i) a disease selected from the group consisting of systemic scleroderma, localized scleroderma, keloids, hypertrophic scars, scars after skin wounding or skin ulceration, skin fibrosis, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints;
ii) a disease selected from the group consisting of systemic scleroderma, localized scleroderma, keloids, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract scars, adipose tissue scars, scars in dystrophic epidermolysis bullosa, and postoperative scars of the skin, digestive tract, peritoneum, muscles, tendons, or joints;
iii) a disease selected from the group consisting of systemic scleroderma, localized scleroderma, keloids, hypertrophic scars, scars after skin wounding or skin ulceration, skin fibrosis, myocardial fibrosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints;
iv) a disease selected from the group consisting of systemic scleroderma, localized scleroderma, keloids, myocardial fibrosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract scars, adipose tissue scars, scars in dystrophic epidermolysis bullosa, and postoperative scars of the skin, digestive tract, peritoneum, muscles, tendons, or joints;
v) a disease selected from the group consisting of systemic scleroderma, localized scleroderma, keloids, hypertrophic scars, scars after skin wounding or skin ulceration, skin fibrosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints;
vi) a disease selected from the group consisting of systemic scleroderma, localized scleroderma, keloids, renal fibrosis, pancreatic fibrosis, pulmonary fibrosis, myelofibrosis, retroperitoneal fibrosis, mesenteric fibrosis, mammary gland fibrosis, cystic fibrosis, digestive tract scars, adipose tissue scars, scars in dystrophic epidermolysis bullosa, and postoperative scars of the skin, digestive tract, peritoneum, muscles, tendons, or joints;
vii) a disease selected from the group consisting of systemic scleroderma, localized scleroderma, keloids, hypertrophic scars, scars after skin wounding or skin ulceration, skin fibrosis, digestive tract fibrosis, glaucoma, age-related macular degeneration, dry eye, chronic GVHD, digestive tract scars, scars in dystrophic epidermolysis bullosa or Kindler syndrome, and postoperative scars of the skin or digestive tract;
viii) a disease selected from the group consisting of systemic scleroderma, localized scleroderma, keloids, digestive tract scars, scars in dystrophic epidermolysis bullosa, and postoperative scars of the skin or digestive tract;
ix) a disease selected from the group consisting of keloids, hypertrophic scars, scars after skin wounding or skin ulceration, skin fibrosis, digestive tract fibrosis, digestive tract scars, scars in dystrophic epidermolysis bullosa or Kindler syndrome, and postoperative scars of the skin or digestive tract;
x) a disease selected from the group consisting of keloids, digestive tract scars, scars in dystrophic epidermolysis bullosa, and postoperative scars of the skin or digestive tract;
xi) a disease selected from the group consisting of keloids, hypertrophic scars, scars after skin wounding or skin ulceration, skin fibrosis, digestive tract fibrosis, digestive tract scars, and postoperative scars of the skin or digestive tract; or
xii) a disease selected from the group consisting of keloids, digestive tract scars, and postoperative scars of the skin or digestive tract.

The present application also provides pharmaceutical compositions for inhibiting fibrosing of tissue, which comprise an HMGB1 fragment peptide comprising the amino acid sequence described in SEQ ID NO: 1.

Furthermore, the present application provides agents for inhibiting fibrosing of tissue, which comprise an HMGB1 fragment peptide comprising the amino acid sequence described in SEQ ID NO: 1. Such agents for inhibiting fibrosing of tissue can be used as reagents in, for example, basic research and clinical research to develop therapeutic medicine for fibrotic diseases. For example, in an experiment to evaluate the effect of a test substance in inhibiting fibrogenesis using a tissue fibrosing model animal, the agent for inhibiting fibrosing of tissue of the present application can be used as a control substance. As used herein, an agent for inhibiting fibrosing of tissue used for non-pharmaceutical purposes are also referred to as "reagent compositions" or "reagents" for inhibiting fibrosing of tissue.

"Fibrosing of tissue" in the present application means fibrogenesis/fibrosing (fibrosis) of any tissue such as skin, cardiac muscle, skeletal muscle, tendon, liver, kidney, pancreas, lung, digestive tract, bladder, bone marrow, peritoneum, mesenterium, mammary gland, adipose tissue, or such.

In one embodiment, a tissue of which fibrosing can be inhibited by a pharmaceutical composition or an agent for inhibiting fibrosing of tissue of the present application is:
i) a tissue selected from the group consisting of skin, skeletal muscle, tendon, liver, kidney, pancreas, lung, digestive tract, bladder, bone marrow, peritoneum, mesenterium, mammary gland, and adipose tissue;
ii) a tissue selected from the group consisting of skin, cardiac muscle, skeletal muscle, tendon, kidney, pancreas, lung, digestive tract, bladder, bone marrow, peritoneum, mesenterium, mammary gland, and adipose tissue;
iii) a tissue selected from the group consisting of skin, skeletal muscle, tendon, kidney, pancreas, lung, digestive tract, bladder, bone marrow, peritoneum, mesenterium, mammary gland, and adipose tissue; or
iv) skin or digestive tract.

The present application also provides pharmaceutical compositions for the treatment of dystrophic epidermolysis bullosa, which comprises an HMGB1 fragment peptide comprising the amino acid sequence described in SEQ ID NO: 1. The present application also provides pharmaceutical compositions for prolonging the lifetime of patients with dystrophic epidermolysis bullosa, which comprises the HMGB1 fragment peptide.

In the present application, "dystrophic epidermolysis bullosa" is a disease in which blisters and intractable skin ulcers are formed due to disruption of adhesion function between the basement membrane and the dermis, caused by a defect or mutation in the Type VII collagen α1 (COL7A1) gene which results in the deficiency or loss of normal function of the Type VII collagen fibers connecting the basement membrane and the dermis. In dystrophic epidermolysis bullosa, epidermolysis, bulla formation and healing are repeated, fibrosing of the dermis worsens and scars are formed, resulting in the adhesion of the fingers. In addition, there are cases in which food intake becomes difficult as stenosis of the digestive tract occurs due to scar formation by similar repeated detachment of the mucosal epithelium in the digestive tract.

In the present application, an HMGB1 fragment peptide comprising the amino acid sequence described in SEQ ID NO: 1 refers to a peptide consisting of a portion of the HMGB1 protein and comprising the amino acid sequence described in SEQ ID NO: 1. Such peptides can be obtained as genetic recombinants by incorporating a DNA encoding the peptide into an appropriate expression system, or they can be synthesized artificially.

In the present application, examples of the HMGB1 protein include, but are not limited to, proteins comprising the amino acid sequence described in SEQ ID NO: 2 and proteins encoded by DNA comprising the nucleotide sequence described in SEQ ID NO: 3.

Examples of the HMGB1 fragment peptide comprising the amino acid sequence described in SEQ ID NO: 1 in the present application include the HMGB1 fragment peptide consisting of the amino acid sequence described in SEQ ID NO: 1.

In the pharmaceutical compositions or agents for inhibiting fibrosing of tissue of the present application, peptides that comprise an amino acid sequence with one or more amino acid residues modified (substitutions, deletions, insertions, or additions) in the amino acid sequence described in SEQ ID NO: 1 and that are functionally equivalent to the HMGB1 fragment peptide comprising the amino acid sequence described in SEQ ID NO: 1 can be used instead of or in conjunction with the HMGB1 fragment peptide comprising the amino acid sequence described in SEQ ID NO: 1. Examples of such peptides include, but are not limited to, the peptides described below as i) to iv), and peptides described below as i) to iv) and having the function of inhibiting fibrosing of tissue:
i) a peptide comprising an amino acid sequence in which one or more amino acids (e.g., one to ten, one to nine, one to eight, one to seven, one to six, one to five, one to four, one to three, or one or two) have been substituted, deleted, inserted, or added in the amino acid sequence described in SEQ ID NO: 1;
ii) a peptide consisting of an amino acid sequence in which one or more amino acids (e.g., one to ten, one to nine, one to eight, one to seven, one to six, one to five, one to four, one to three, or one or two) have been substituted, deleted, inserted, or added in the amino acid sequence described in SEQ ID NO: 1;
iii) a peptide comprising an amino acid sequence having about 80% or more, for example, about 85% or more, about 90% or more, about 91% or more, about 92% or more, about 93% or more, about 94% or more, about 95% or more, about 96% or more, about 97% or more, about 98% or more, or about 99% or more sequence identity with the amino acid sequence described in SEQ ID NO: 1; and
iv) a peptide consisting of an amino acid sequence having about 80% or more, for example, about 85% or more, about 90% or more, about 91% or more, about 92% or more, about 93% or more, about 94% or more, about 95% or more, about 96% or more, about 97% or more, about 98% or more, or about 99% or more sequence identity with the amino acid sequence described in SEQ ID NO: 1.

An effective amount of a peptide of the present application or a pharmaceutical composition comprising the peptide (hereafter referred to as a peptide or such) is administered to a subject for the treatment or prevention of a disease or symptom described herein.

An effective amount as used herein refers to an amount sufficient for the treatment of a disease or symptom as described herein. Treatment in the present application includes, but is not limited to, alleviation, delay, blockade, improvement, remission, cure, complete cure, and such.

Subjects in the present application include, without limitation, mammals, birds, fish, and such. Mammals include, but are not limited to, humans and non-human animals, for example, humans, mice, rats, monkeys, pigs, dogs, rabbits, hamsters, guinea pigs, horses, sheep, whales, and such. In the present application, the term "subject" is used interchangeably with "patient", "individual", and "animal".

There is no limitation in the site of administration of the peptide or such of the present application, and the peptide or such of the present application can exert a fibrogenesis-inhibiting effect, even if it is administered to any site such as a site where production and/or deposition of more collagen fibers than the normal site is observed or a vicinity thereof, or a site different therefrom (for example, a site distant from the site where production and/or deposition of more collagen fibers than the normal site is observed, or a site that belongs to a tissue different from the tissue that has the site where production and/or deposition of more collagen fibers than the normal site is observed).

Methods of administering the peptide or such of the present application include, but are not limited to, oral administration and parenteral administration including intravascular (intra-arterial, intravenous, and such), intramuscular, subcutaneous, intradermal, intraperitoneal, nasal, pulmonary, and transdermal administrations. Also, the peptide or such of the present application can be administered systemically or locally (e.g., subcutaneously, intradermally, or to the skin surface, eyeball or palpebral conjunctiva, nasal mucosa, oral and gastrointestinal mucosa, vaginal and endometrial mucosa, or injured site) by injection administration, for example, intravenous injection, intramuscular injection, intraperitoneal injection, and subcutaneous injection.

Further, the administration method can be appropriately selected according to the age and symptoms of the patient. When administering the peptides of the present application, for example, the dosage can be selected from the range of 0.0000001 mg to 1000 mg per kilogram of body weight per administration. Alternatively, the dosage can be selected, for example, from the range of 0.00001 to 100000 mg/body per patient. Also when a cell secreting a peptide of the present application or a gene therapy vector into which a DNA encoding the peptide has been inserted is administered, administration can be performed so that the amount of the peptide is within the above range. However, the pharmaceutical compositions in the present application are not limited to these doses.

Pharmaceutical compositions of the present application can be formulated according to conventional methods (e.g., Remington's Pharmaceutical Science, latest edition, Mark Publishing Company, Easton, U.S.A.), and may contain pharmaceutically acceptable carriers and additives together. Examples include, but are not limited to, surfactants, excipients, coloring agents, perfumes, preservatives, stabilizers, buffers, suspending agents, isotonizing agents, binding agents, disintegrants, lubricants, fluidity-promoting agents, and flavoring agents, and other commonly used carriers can be used as appropriate. Specific examples include, light anhydrous silicic acid, lactose, crystalline cellulose, mannitol, starch, carmellose calcium, carmellose sodium, hydroxypropyl cellulose, hydroxypropyl methylcellulose, polyvinylacetal diethylaminoacetate, polyvinylpyrrolidone, gelatin, medium-chain fatty acid triglycerides, polyoxyethylene hydrogenated castor oil 60, white sugar, carboxymethyl cellulose, cornstarch, inorganic salts.

All prior art documents cited herein are incorporated herein as references.

The present invention is further illustrated by, but not limited to, the examples below.

### Examples

### [Example 1] Inhibitory effect of the HMGB1 peptide (1-44) on forelimb adhesion in epidermolysis bullosa model mice

### (1) Materials and methods

### i) Animals

Epidermolysis bullosa model mice which have a mutant allele of the Type VII collagen-α1 (Col7a1) gene homozygously (described in Fritsch, et al. J Clin Invest. 2008 May;118(5):1669-79 as Col7a1^{flNeo/flNeo} mice, and hereafter referred to as "129SV/colVII homo mice" herein) were obtained from Professor Leena Bruckner-Tuderman of the University of Freiburg (Germany). Fifteen of the mice (males) were acclimated in the breeding room for two weeks, divided into two groups so that the adhesion scores described below were comparable, and administration of the test substance was begun on the following day (weeks of age at the start of administration: 5-6 weeks old). One animal in the HMGB1 peptide (1-44)-treated group died due to a mistake in the anesthetic procedure at the time of adhesion score assessment one week after the start of administration of the test substance, and it was excluded from the study. Therefore, the group composition was as follows.

### Group composition:

| | |
|---|---|
| Vehicle group | 7 animals |
| HMGB1 peptide (1-44) group | 7 animals |

### ii) Production of the test substance

A peptide consisting of amino acid residues 1-44 (SEQ ID NO: 1) of the human-derived HMGB1 protein was chemically synthesized by the solid-phase method. This peptide is called the HMGB1 peptide (1-44).

### iii) Administration of the test substance

Vehicle (isotonic sodium chloride solution, "Otsuka Normal Saline" manufactured by Otsuka Pharmaceutical Factory) or the HMGB1 peptide (1-44) (concentration of 0.2 mg/mL) was intravenously administered via the tail vein at a volume of 50 µL, respectively, using Myjector (Terumo) over approximately 2-3 seconds. The test substance was administered from the day after grouping, twice a week with at least one day administration interval, over four weeks for a total of eight doses.

### iv) Evaluation of adhesion scores for forelimb fingers

The degree of adhesion of forelimb fingers in the mice was evaluated macroscopically under light isoflurane anesthesia, the day before the start of administration of the test substance and once a week for one to eight weeks after the start of administration for a total of nine times according to the scores below. The left and right forelimbs were separately evaluated for Items A to D below, and the individual scores were added up to give a value as the adhesion score.

Adhesion score = sum of the points for which Items A through D were separately evaluated for the left and right forelimbs
A. Adhesion between the second and third fingers

| | |
|---|---|
| 0 point: | No adhesion |
| 1 point: | Partial adhesion to the distal interphalangeal (DIP) joint |
| 2 points: | Adhesion beyond the DIP joint |

B. Adhesion between the third and fourth fingers

| | |
|---|---|
| 0 point: | No adhesion |
| 1 point: | Partial adhesion to the DIP joint |
| 2 points: | Adhesion beyond the DIP joint |

C. Adhesion of the fifth finger

| | |
|---|---|
| 0 point: | No adhesion |
| 1 point: | Partial adhesion |
| 2 points: | Complete adhesion to palm |

D. Presence or absence of nails on the second, third, and fourth fingers

| | |
|---|---|
| 0 point: | All three have nails |
| 1 point: | One or two fingers with missing nails |
| 2 points: | All three fingers have no nails |

### v) Handling of dead mice

One specimen which died at the time of evaluation of the adhesion score due to a mistake in the anesthetic procedure was excluded from the analysis and not reflected in the results, since it was immediately after the start of administration of the test substance. One specimen in the vehicle group died each at seven and eight weeks after the start of treatment. The cause of death was considered to be digestive tract adhesion due to disease progression. The adhesion scores of these two animals were reflected in the results, and the adhesion scores after death were regarded as missing values.

### vi) Statistical analysis

Statistical analysis was performed using GraphPad Prism7 (GraphPad Software, Inc., La Jolla, CA). Data for the adhesion scores showed interactions when two-way analysis of variance with repeat measurements was performed on data of up to six weeks which do not have missing values, and thus the Mann-Whitney's U test was performed at each time point of assessment. It was deemed as statistically significant difference when p<0.05.

### (2) Results

Fig. 1 shows adhesion scores on the mouse forelimbs for the day before the start of administration and one to eight weeks after the start of administration in the vehicle group and the HMGB1 peptide (1-44) group in this study, and Fig. 2 shows photographs of the left and right forelimbs at six weeks after the start of administration. Regarding adhesion of the forelimbs, although some slight adhesion was observed at six weeks of age at the start of administration, the disease state progressed with age; and in the vehicle group, partial adhesion or adhesion beyond the DIP joint was observed in almost all fingers except for two animals at six weeks (12 weeks of age) after the start of administration. On the other hand, in the HMGB1 peptide (1-44) group, the degree of increase of the adhesion score was suppressed compared with the vehicle group, and the adhesion score at four to eight weeks (10 to 14 weeks of age) after the start of administration was about half of that in the vehicle group, which was statistically significantly lower (Fig. 1). Mice in both the vehicle group and HMGB1 peptide (1-44) group maintained similar body weights from the start of treatment to Week 8, and there was no significant difference between the groups.

Finger adhesions are observed in the 129SV/colVII homo mice with age in weeks, as in patients with epidermolysis bullosa. Epidermolysis bullosa in the 129SV/colVII homo mice is classified as dystrophic epidermolysis bullosa caused by a mutation in the Col7a1 gene. In this disorder type, Type VII collagen is not produced normally, and blisters are formed by failure of the adhesion function between the basement membrane and the dermis, and adhesion between the fingers and toes is thought to progress due to repeated scarring of blisters in the limbs (Fritsch, et al., supra). In this study, intravenous administration of the HMGB1 peptide (1-44) was shown to inhibit adhesion of the forelimb fingers of the 129SV/colVII homo mice. These results indicate that the HMGB1 peptides have an effect of inhibiting fibrosing of the skin tissue.

### [Example 2] Inhibitory effect of the HMGB1 peptide (1-44) on fibrosing and scarring of intestinal tract in the epidermolysis bullosa mouse model

### (1) Materials and methods

### i) Animals

The 129SV/colVII homo mice described in Example 1 (6 males) were acclimated in the breeding room for two weeks, and divided into the vehicle group (3 animals) and HMGB1 peptide (1-44) treatment group (3 animals), and then administration of the test substance was begun (weeks of age at the start of administration: 5-6 weeks old).

### ii) Test substance

As in Example 1, the chemically synthesized HMGB1 peptide (1-44) was used as the test substance.

### iii) Administration of the test substance

Vehicle (isotonic sodium chloride solution, "Otsuka Normal Saline" manufactured by Otsuka Pharmaceutical Factory) or the HMGB1 peptide (1-44) (concentration of 0.2 mg/mL) was administered via the tail vein at a volume of 50 µL, respectively. The test substance was administered from the day after grouping, twice a week with at least one day administration interval, over four weeks for a total of eight doses.

### iv) Evaluation of the effect of the test substance on the small intestine tissue

Mice continued to be reared even after administration of the test substance was completed. The small intestine was sampled when mice of the vehicle group showed abdominal distention, and at the same time, the small intestine was sampled from mice of the HMGB1 peptide (1-44)-treated group. Tissue sections were then prepared from the collected small intestine samples and stained with hematoxylin-eosin (HE) and Masson's-Trichrome (MT).

### (2) Results

Mice of the vehicle group (n = 3) each showed abdominal distention at Week 7 (13 weeks of age), Week 10 (16 weeks of age), and Week 11 (17 weeks of age) after the start of treatment, and thus, the small intestine was sampled for HE staining and MT staining at those time points. In line with the above, the small intestine of mice in the HMGB1 peptide (1-44)-treated group was also sampled from one animal each at Week 7 (13 weeks of age), Week 10 (16 weeks of age), and Week 11 (17 weeks of age) after the start of administration, and HE staining and MT staining were performed.

Representative images of the HE staining results of the small intestine of mice in the vehicle group and HMGB1 peptide (1-44) group are shown in Fig. 3. In the vehicle group, intestinal stenosis, filling of gas, and loss of villi were observed in all three animals, whereas none of the animals in the HMGB1 peptide (1-44)-treated group had these symptoms. In the small intestine of the vehicle group, thickening of the intestinal wall due to scarring was observed near the site where stenosis and distention occurred, whereas no thickening of the intestinal wall was observed in the HMGB1 peptide (1-44)-treated group. From the results of MT staining, notable collagen fibril deposition in the small-intestinal wall of the vehicle group was observed, whereas collagen fibril deposition was almost not seen in the HMGB1 peptide (1-44)-treated group (Fig. 4). These results indicate that the HMGB1 peptide has an inhibitory effect on the fibrosing and scarring of the small intestinal tissue.

### [Example 3] Prolonged survival of epidermolysis bullosa model mice by HMGB1 peptide (1-44)

### (1) Materials and methods

### i) Animals

The 129SV/colVII homo mice described in Example 1 (males) were acclimated in the breeding room for two weeks, and divided into the vehicle group and HMGB1 peptide (1-44) treatment group, and then administration of the test substance was begun (weeks of age at the start of administration: 5-6 weeks old (body weight 5 to 8 g)).

### ii) Test substance

As in Example 1, the chemically synthesized HMGB1 peptide (1-44) was used as the test substance.

### iii) Administration of the test substance

Vehicle (isotonic sodium chloride solution, "Otsuka Normal Saline" manufactured by Otsuka Pharmaceutical Factory) or the HMGB1 peptide (1-44) (concentration of 0.2 mg/mL) was administered via the tail vein at a volume of 50 µL per administration, respectively. The test substance was administered from the day after grouping, twice a week with at least one day administration interval, over four weeks for a total of eight doses.

### iv) Assessment of survival time

Mice continued to be reared even after administration of the test substance was completed until they died, and the survival time of each animal in the vehicle group (10 animals) and HMGB1 peptide (1-44)-treated group (10 animals) was recorded. In both groups, for some of the animals, observation was terminated while they were still alive as it became difficult to continue stable breeding and feeding due to long-term shutdown of the animal breeding facility. Specifically, observation of one animal in the vehicle group was terminated at Week 13.14, while in the HMGB1 peptide (1-44)-treated group, observation was terminated for one animal at Week 13.14, one animal at Week 18.57, two animals at Week 21.57, and one animal at Week 36.57.

### (2) Results

Survival curves based on the survival time of each animal in the vehicle group and HMGB1 peptide (1-44)-treated group are shown in Fig. 5 (start of administration of the test substance was Week 0). It was shown that administration of the HMGB1 peptide (1-44) significantly prolongs the survival time of the epidermolysis bullosa model mice.

### [Example 4] Effect of HMGB1 peptide (1-44) treatment on the full-thickness skin defect-type ulcer mouse model

### (1) Materials and methods

### i) Animals

C57BL/6 male mice (C57BL/6JJcl; microbial-grade SPF) were purchased from CLEA Japan, Inc. at 7-8 weeks of age and used after five days or more of acclimatization in the animal breeding room.

### ii) Generation of skin ulcers

On the day before the full-thickness skin defect operation for the generation of lesions and ulcers, dorsal hair of the experimental animals was removed under isoflurane anesthesia (anesthetic machine (MK-A110D, Muromachi Kikai Co., Ltd.), anesthetic condition: 4.0 % at the time of introduction and 2.0 % for maintenance; flow rate: 1.5 L/minute). In ulcer generation, after the dorsal skin was disinfected with Isodine or alcohol under isoflurane anesthesia, an ulcer was produced by scooping out the skin using a biopsy trepan (5 mm in diameter), and the animal was returned to the cage and reared. For postoperative management, the ulcer area was wrapped and protected with a dressing. For hygiene management and observation of postoperative conditions, sheets, dressings, and bandages were changed every 72-96 hours until approximately two weeks passed when the wound closed spontaneously. In addition, the risk of infection was minimized by keeping the lesions in a sterile condition during the experiment.

### iii) Grouping

Epithelial contraction was suppressed with a silicone sheet after the skin ulcer was created, and under such condition, photographs were taken from the rooftop side at about 20 cm from the dorsal skin so that the shape and diameter of the ulcer were well visualized. Based on the photographs, mice with 2 points or higher on the skin ulcer evaluation standards shown below were adopted as those that may ensure a microenvironment in which contraction inhibition of epithelial sheet from the surrounding area and granulation occur uniformly. The mice were divided into the vehicle (saline) treatment group and HMGB1 peptide (1-44) treatment group, with nine mice per group.

### <Skin ulcer evaluation standards>

3 points: Longitudinal diameter (4.5-5.5 mm) and transverse diameter (4.5-5.5 mm) of a circle, nearly complete circle (equal space of gap with sheet)
2 points: Longitudinal diameter (4.5-5.5 mm) and transverse diameter (4.5-5.5 mm) of a circle fulfilled, but not a complete circle.
1 point: Either longitudinal diameter (4.5-5.5 mm) or transverse diameter (4.5-5.5 mm) of a circle fulfilled, ellipse to circle
0 point: Other than the above, and no full-thickness defect recognized (fascia not observed)

### iv) Test substance

As in Example 1, the chemically synthesized HMGB1 peptide (1-44) was used as the test substance.

### v) Administration of the test substance

The test substance was administered eight times in total, starting at 3 hours after ulcer creation and then repeated on the 4^{th}, 8^{th}, 11^{th}, 15^{th}, 18^{th}, 22^{nd}, and 25^{th} days. The dose of the HMGB1 peptide (1-44) was set to 1.5 mg/kg/day, and a solution containing the diluted vehicle was filled into 1 mL syringes and administered via the tail vein using a 30G needle.

### vi) Sampling of ulcer sites

On the 28^{th} day after skin ulcer creation, mice were placed under the influence of isoflurane anesthesia and euthanized by carrying out cervical dislocation, and the skin in the ulcer site and its surrounding area was quickly excised and collected while preventing protein degeneration of the samples on ice. The collected skin samples were placed in 10 % Formalin (Wako) after removal of excess subcutaneous fat with forceps and fixated overnight at 4 degrees while shaking on ice with a shaker. After fixation, the skin was thoroughly washed with PBS, and the specimens were trimmed and made into paraffin blocks with an automated embedder (Thermo Scientific), while attention was paid on the tissue polarity of the skin-specific craniocaudal/left-right axis. Then, they were sliced (5 µm) with a rotatory microtome (Thermo Scientific), fixed on a slide glass (Matsunami Glass), and subjected to Masson's-Trichrome (MT) staining.

### vii) Qualitative/quantitative evaluation of histological changes in the ulcer sites by Masson's-Trichrome (MT) staining

In order to measure the area occupied by collagen fibers in the regenerated and healed ulcer sites, Masson's-Trichrome staining was performed on paraffin slices (5 µm) and the amount of collagen in the dermis/scar sites was determined by stainability. The details were taken by first photographing the ulcer site (center) and the margin (surrounding normal region) of a total of six sections per animal by microscopy (Keyence BZ-X710). The amount of collagen in the regions was then measured using an image analysis software (BZ-X Analyzer). The calculations were performed using values in the margin region contiguous to each center region to compensate for the inter-individual variation of the collagen content at the dermis/scar sites in the skin-specific hair cycle. Namely, [corrected percentage by area of collagen-positive region directly below the ulcer site] = [percentage by area of collagen-positive region in the center region/percentage by area of collagen-positive region in the margin region].

### viii) Statistical processing

Measured values obtained by the above analysis or the corrected calculated values were analyzed/determined after statistical values were calculated using mainly a statistical analysis software (statcel-3). Specifically, (1) test of normality (test with Fisher's skewness/kurtosis coefficients; P>0.05 is considered to be normal distribution), (2) test of variance (Levene's test; P>0.05 is considered to be of equal variance), and (3) test of rejection (Grubb's test; individual values with P<0.05 are excluded as outliers) were carried out as preliminary tests. When the results obtained in (1) and (2) showed normal distribution, a t test with the average value between the groups (Student (equal variance) or Welch test (unequal variance)) was performed, and when normal distribution was not shown, a non-parametric test with the rank between the groups (Mann Whitney's test) was performed, and the difference was judged to be significant with P<0.05.

### (2) Results

Skin was harvested 28 days after ulcer creation and Masson's-Trichrome staining was performed to evaluate histological changes in the mouse skin ulcer sites between the treatment groups. Representative images of the staining results are shown in Fig. 6, and the analysis results for the above-calculated [corrected percentage by area of collagen-positive region directly below the ulcer site] are shown in Fig. 7. In the margin region of the vehicle-treated group, collagen fibers have irregular alignment orientation characteristics and there is abundant collagen fiber composition, and thus, a strong wave-shape signal is detected. In contrast, in the center region, which is the center of ulcer, collagen fibers are newly synthesized by a population of cells with high collagen fiber-synthesizing ability represented by the myofibroblast population, the fibers have regular alignment orientation characteristics and are less in quantity, and thus, a linear and weak signal is detected (Fig. 6). Compared with the vehicle-treated group, the signal of collagen fibers in the center region of HMGB1 peptide (1-44)-treated group tended to be weak (Fig. 6), and significant suppression was observed when quantitative signal strength and area ratios were calculated (*p<0.05, Mann Whitney's test) (Fig. 7). The results indicate that the HMGB1 peptide has the effect of inhibiting fibrosing of skin tissue.

There were no notable differences in the body weight changes in mice of any group during the implementation period of this study (from the start of the operation (Day 1) to the time of skin collection (Day 28)), and there were no statistically significant changes between the groups (p>0.05, Repeated one-way ANOVA).

### Industrial applicability

Dystrophic epidermolysis bullosa is a disease caused by genetic abnormalities, and thus, its treatment method includes a method of allotransplanting pluripotent stem cells from a normal person, or a method of transplanting iPS cells derived from the patient himself to the patient after repairing the mutation of the COL7A1 gene by genome editing or such. However, the former method has the problem of rejection, and the latter method has a problem that risk of canceration by the off-target action of genome editing cannot be eliminated.

This time, the present inventors surprisingly discovered for the first time that administration of the HMGB1 fragment peptide, which is neither allogeneic transplantation of cells nor gene therapy, ameliorates the symptoms of dystrophic epidermolysis bullosa. As a result of investigating the mechanism of the therapeutic effects, it was found that the HMGB1 fragment peptide inhibits fibrosing of tissue.

The HMGB1 fragment peptide inhibited collagen deposition in the intestinal tract tissues of the model mice of dystrophic epidermolysis bullosa, which is a hereditary disorder. In addition, the HMGB1 fragment peptide also inhibited collagen deposition in the skin tissues in the skin ulcer model in which physical skin defects were created in normal mice that do not have genetic abnormalities. Thus, the HMGB1 fragment peptide was recognized to have effects of inhibiting deposition of collagen, which is an extracellular matrix protein, and suppressing fibrosing of tissue in different models and tissues. Therefore, pharmaceutical compositions comprising the HMGB 1 fragment peptide of the present application may be useful in the treatment of fibrosis and scars of any organ or tissue with collagen deposition.

## Claims

1. A pharmaceutical composition for treating a fibrotic disease, which comprises the substance described in any of (a) to (c) below:
(a) an HMGB1 fragment peptide comprising the amino acid sequence described in SEQ ID NO: 1;
(b) a peptide comprising an amino acid sequence in which one or more amino acids have been substituted, deleted, inserted, or added in the amino acid sequence described in SEQ ID NO:1; and
(c) a peptide comprising an amino acid sequence having about 80 % or more sequence identity with the amino acid sequence described in SEQ ID NO: 1.

2. The pharmaceutical composition of claim 1, wherein the fibrotic disease is selected from the group consisting of systemic scleroderma, localized scleroderma, keloids, hypertrophic scars, scars after skin wounding or skin ulceration, skin fibrosis, myocardial fibrosis, liver fibrosis, liver cirrhosis, renal fibrosis, pancreatic fibrosis, pulmonary fibrosis, myelofibrosis, retroperitoneal fibrosis, mesenteric fibrosis, mammary gland fibrosis, cystic fibrosis, digestive tract fibrosis, adipose tissue fibrosis, glaucoma, age-related macular degeneration, dry eye, chronic GVHD, digestive tract scars, adipose tissue scars, scars in dystrophic epidermolysis bullosa or Kindler syndrome, and postoperative scars of the skin, digestive tract, peritoneum, muscles, tendons, or joints.

3. A pharmaceutical composition for inhibiting fibrosing of a tissue, which comprises the substance described in any of (a) to (c) below:
(a) an HMGB1 fragment peptide comprising the amino acid sequence described in SEQ ID NO: 1;
(b) a peptide comprising an amino acid sequence in which one or more amino acids have been substituted, deleted, inserted, or added in the amino acid sequence described in SEQ ID NO:1; and
(c) a peptide comprising an amino acid sequence having about 80 % or more sequence identity with the amino acid sequence described in SEQ ID NO: 1.

4. An agent for inhibiting fibrosing of a tissue, which comprises the substance described in any of (a) to (c) below:
(a) an HMGB1 fragment peptide comprising the amino acid sequence described in SEQ ID NO: 1;
(b) a peptide comprising an amino acid sequence in which one or more amino acids have been substituted, deleted, inserted, or added in the amino acid sequence described in SEQ ID NO:1; and
(c) a peptide comprising an amino acid sequence having about 80 % or more sequence identity with the amino acid sequence described in SEQ ID NO: 1.


SEQUENCE LISTING

<110>  Genomix Co., Ltd.
       Osaka University

<120>  An agent for the treatment of fibrotic disease

<130>  G6-A1701P

<150>  JP 2017-076600
<151>  2017-04-07

<160>  3

<170>  PatentIn version 3.5

<210>  1
<211>  44
<212>  PRT
<213>  Artificial sequence

<220>
<223>  An artificially synthesized peptide sequence

<400>  1

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Lys
        35                  40

<210>  2
<211>  215
<212>  PRT
<213>  Homo sapiens

<400>  2

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Lys Cys Ser Glu Arg
        35                  40                  45

Trp Lys Thr Met Ser Ala Lys Glu Lys Gly Lys Phe Glu Asp Met Ala
    50                  55                  60

Lys Ala Asp Lys Ala Arg Tyr Glu Arg Glu Met Lys Thr Tyr Ile Pro
65                  70                  75                  80

Pro Lys Gly Glu Thr Lys Lys Lys Phe Lys Asp Pro Asn Ala Pro Lys
                85                  90                  95

Arg Pro Pro Ser Ala Phe Phe Leu Phe Cys Ser Glu Tyr Arg Pro Lys
            100                 105                 110

Ile Lys Gly Glu His Pro Gly Leu Ser Ile Gly Asp Val Ala Lys Lys
        115                 120                 125

Leu Gly Glu Met Trp Asn Asn Thr Ala Ala Asp Asp Lys Gln Pro Tyr
    130                 135                 140

Glu Lys Lys Ala Ala Lys Leu Lys Glu Lys Tyr Glu Lys Asp Ile Ala
145                 150                 155                 160

Ala Tyr Arg Ala Lys Gly Lys Pro Asp Ala Ala Lys Lys Gly Val Val
                165                 170                 175

Lys Ala Glu Lys Ser Lys Lys Lys Lys Glu Glu Glu Glu Asp Glu Glu
            180                 185                 190

Asp Glu Glu Asp Glu Glu Glu Glu Glu Asp Glu Glu Asp Glu Asp Glu
        195                 200                 205

Glu Glu Asp Asp Asp Asp Glu
    210                 215

<210>  3
<211>  648
<212>  DNA
<213>  Homo sapiens

<400>  3
atgggcaaag gagatcctaa gaagccgaga ggcaaaatgt catcatatgc attttttgtg      60

caaacttgtc gggaggagca taagaagaag cacccagatg cttcagtcaa cttctcagag     120

ttttctaaga agtgctcaga gaggtggaag accatgtctg ctaaagagaa aggaaaattt     180

gaagatatgg caaaagcgga caaggcccgt tatgaaagag aaatgaaaac ctatatccct     240

cccaaagggg agacaaaaaa gaagttcaag gatcccaatg cacccaagag gcctccttcg     300

gccttcttcc tcttctgctc tgagtatcgc ccaaaaatca aaggagaaca tcctggcctg     360

tccattggtg atgttgcgaa gaaactggga gagatgtgga ataacactgc tgcagatgac     420

aagcagcctt atgaaaagaa ggctgcgaag ctgaaggaaa aatacgaaaa ggatattgct     480

gcatatcgag ctaaaggaaa gcctgatgca gcaaaaaagg gagttgtcaa ggctgaaaaa     540

agcaagaaaa agaaggaaga ggaggaagat gaggaagatg aagaggatga ggaggaggag     600

gaagatgaag aagatgaaga tgaagaagaa gatgatgatg atgaataa 648